# EUROPEAN PATENT APPLICATION

(11) **EP 3 248 639 A1**
(43) Date of publication of application: **29.11.2017**
(21) Application number: 16740151.2
(22) Date of filing: 19.01.2016
(51) Int. Cl.: A61M 5/34, A61J 15/00, A61M 39/02

(54) **MEDICAL LIQUID-COLLECTION TIP, LIQUID-COLLECTION NOZZLE, AND INJECTOR SET**

(30) Priority: 19.01.2015 JP 2015008088
(71) Applicant: JMS. Co., Ltd., Hiroshima 730-8652 (US)
(72) Inventor: YUKI, Takehiko, Hiroshima 730-8652 (JP); UEHARA, Yasumasa, Hiroshima 730-8652 (JP); KUNISHIGE, Takahiko, Hiroshima 730-8652 (JP); NAKANO, Kiyomi, Hiroshima 730-8652 (JP); UEHARA, Megumi, Hiroshima 730-8652 (JP); TAKIMOTO, Kazuhiko, Hiroshima 730-8652 (JP); ISHIDA, Miki, Hiroshima 730-8652 (JP)
(74) Representative: Piésold, Alexander James
(86) International application number: PCT/JP2016/051411
(87) International publication number: WO 2016/117544

(57) **Abstract**

The medical liquid collection tip (1) includes a male member (11) that is to be inserted into a tube end (154) of an injector (150) at one end, and includes a liquid collection needle (20) at the other end. A flow path (8) penetrates through the tip (1) in a lengthwise direction thereof and causes the male member (11) and the liquid collection needle (20) to be in communication. The outer circumferential surface (12) of the male member (11) is a male tapered surface with an outer diameter that becomes smaller as the leading end is approached, and is configured to be fluid-tightly connected to the female tapered surface of the tube end (154).

## Description

### Technical Field

The present invention relates to a medical liquid collection tip and a liquid collection nozzle that are used by being mounted on a tube end at the tip of an injector. Also, the present invention relates to an injector set including the medical liquid collection tip or liquid collection nozzle and an injector.

### Background Art

Enteral nutrition therapy is known as a method for non-orally administering nutrition and medicine to a patient. In enteral nutrition therapy, a nasal catheter inserted into the stomach or duodenum through the nasal cavity of a patient, or a PEG (percutaneous endoscopic gastrostomy) catheter inserted into a gastric fistula formed in the stomach of a patient is used. A liquid such as nutrients, liquid food (commonly known as "enteral nutrients"), or medicine is administered to the patient via the nasal catheter or the PEG catheter (hereinafter collectively referred to as "catheter"). At the time of administering the liquid to the patient, a connector (hereinafter referred to as a "patient-side connector") provided on an upstream end of a catheter inserted into the patient, or provided on an upstream end of a flexible tube (commonly known as an "extension tube") that is connected to the catheter, and a connector (hereinafter referred to as a "container-side connector") connected to a container storing the liquid, or to a tube connected to the container, are connected. Conventionally, a female connector has been used as the patient-side connector and a male connector has been used as the container-side connector (e.g., see Patent Document 1).

In many cases, the patient into which the catheter is inserted cannot directly swallow medicine such as a tablet through the mouth. A "simple suspension method" is known as a method for administering medicine to the patient in such a case. The simple suspension method is performed using the following procedure. First, medicinal liquid obtained by disintegrating a tablet in lukewarm water or the like is formed in a container. Next, the medicinal liquid is suctioned into the injector (syringe). Next, the tube end of the injector is connected to the patient-side connector, and the medicinal liquid is administered to the patient via the catheter.

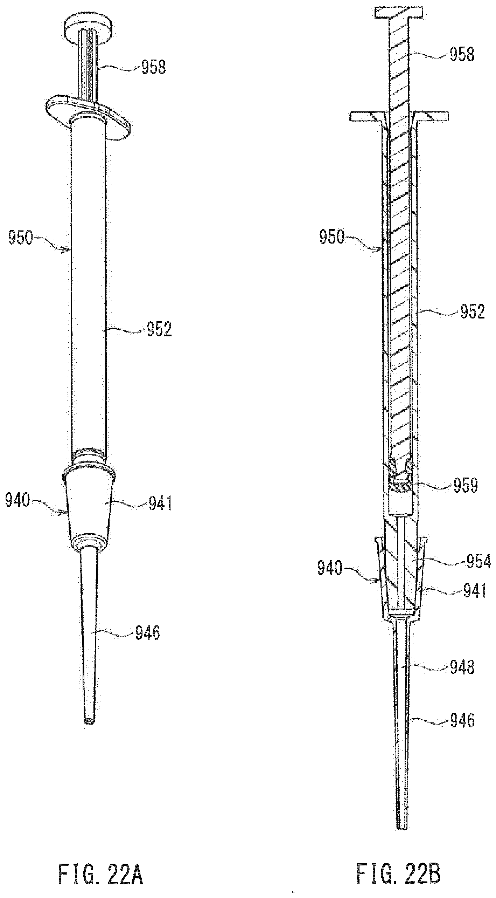

With the simple suspension method, it is necessary to connect the tube end of the injector to the patient-side connector. FIG. 22A is a perspective view of an injector 950 provided with a conventional liquid collection tip 940 used in the simple suspension method, and FIG. 22B is a cross-sectional view of the injector 950 provided with the liquid collection tip 940. Similarly to a general-purpose syringe, the injector 950 includes a barrel (outer tube) 952 and a plunger 958 that moves into and out of the barrel 952. The outer circumferential surface of the tube end (nozzle) 954 of the barrel 952 is a tapered surface (male tapered surface) with an outer diameter that becomes smaller as the leading end is approached. The liquid collection tip 940 is detachably mounted on the tube end 954. The liquid collection tip 940 includes, at one end, a base end portion 941 that is to be fluid-tightly connected to the tube end 954, and includes a liquid collection needle 946 at the other end. A flow path 948 that penetrates through the liquid collection tip 940 causes the base end portion 941 and the liquid collection needle 946 to be in communication. In the simple suspension method, in a state in which the liquid collection tip 940 is mounted on the injector 950 as shown in FIGS. 22A and 22B, the medicinal liquid is suctioned into the barrel 952 while the leading end of the liquid collection needle 946 is immersed in the medicinal liquid. Thereafter, the liquid collection tip 940 is removed from the injector 950, the tube end 954 is inserted into a patient-side connector (female connector), and the medicinal liquid in the barrel 952 is administered to the patient.

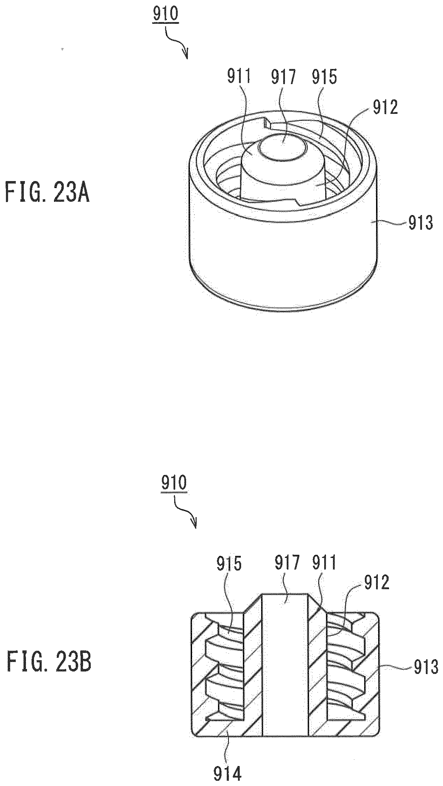

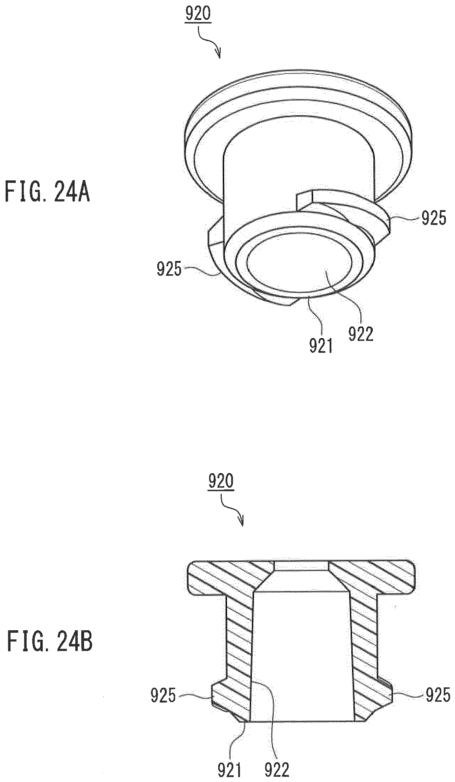

Incidentally, in recent years, consideration has been given to internationally standardizing, as International Standard ISO 80369-3 regarding nutritional medical devices, a male connector 910 shown in FIGS. 23A and 23B as a patient-side connector and a female connector 920 shown in FIGS. 24A and 24B as a container-side connector in order to prevent misconnection with a connector to be used in a field other than enteral nutrition.

The male connector (patient-side connector) 910 shown in FIGS. 23A and 23B includes a cylindrical male member 911 and an outer tube 913 that surrounds the male member 911. The male member 911 and the outer tube 913 are joined via a bottom plate 914 that protrudes in the form of a flange along the radial direction from the base end portion of the male member 911. The outer circumferential surface 912 of the male member 911 is a tapered surface (a so-called male tapered surface) with an outer diameter that becomes smaller as the leading end is approached. A flow path 917 that penetrates through the male member 911 is formed on the male member 911 along the lengthwise direction thereof. A female screw 915 is formed on the inner circumferential surface of the outer tube 913, which opposes the male member 911.

On the other hand, the female connector (container-side connector) 920 shown in FIGS. 24A and 24B includes a cylindrically-shaped tubular portion (female lure) 921 into which the male member 911 is inserted. The inner circumferential surface 922 of the tubular portion 921 is a tapered surface (so-called female tapered surface) with an inner diameter that becomes larger as the leading end is approached. A spiral projection (male screw) 925 is formed on the outer circumferential surface of the tubular portion 921.

The male connector 910 and the female connector 920 are connected due to the male member 911 being inserted into the tubular portion 921 and the female screw 915 and the spiral protrusion 925 being screwed together. The outer circumferential surface 912 of the male member 911 and the inner circumferential surface 922 of the tubular portion 921 are tapered surfaces with the same diameter and tapering angle, and therefore both come into fluid-tight surface contact with each other. The female screw 915 and the spiral projection 925 that are screwed together constitute a lock mechanism for locking the connected state of the male connector 910 and the female connector 920. The male connector 910 and the female connector 920 provide a connection with excellent fluid-tightness (a property of not allowing the liquid to leak from the portion at which the male connector and the female connector are connected, even if pressure is applied to the liquid) and connection strength (a property according to which the connected male connector and female connector do not separate even if a pulling force is applied thereto).

In the case where the patient-side connector is the male connector 910 shown in FIGS. 23A and 23B, the tube end 954 of the injector 950 shown in FIGS. 22A and 22B cannot be connected to the male connector 910. The tube end of the injector that is used in the simple suspension method needs to include the female connector 920 shown in FIGS. 24A and 24B in order to be able to connect to the male connector 910.

### Citation List

### Patent Documents

Patent Document 1: WO 2008/152871

### Disclosure of Invention

### Problem to be Solved by the Invention

If the tube end of the injector includes the female connector 920, the medicinal liquid attaches to the inner circumferential surface of the outer tube 913 of the male connector (patient-side connector) 910 when the female connector 920 is connected to the male connector 910 in the state in which the medicinal liquid is attached to the outer circumferential surface (in particular, the spiral projection 925) of the tubular portion 921.

As shown in FIGS. 23A and 23B, the female screw 915 is formed on the inner circumferential surface of the outer tube 913 of the male connector 910. When the medicinal liquid attaches to the troughs of the female screw 915, it is difficult to remove the medicinal liquid by wiping. If the male connector 910 is provided on the upstream-side end of the catheter inserted in the patient, the male connector 910 continues to be left in the patient along with the catheter for a long time in some cases. For example, a PEG catheter is commonly replaced every 1 to 3 months. If the medicinal liquid thus continues to be attached to the male connector 910 for a long time, the male connector 910 can reach an unhygienic state. Then, there is a possibility that bacteria will eventually reproduce in the male connector 910 and the bacteria will enter the body of the patient and cause serious complications.

In order to prevent the male connector 910 from reaching an unhygienic state such as that described above, it is necessary to prevent the medicinal liquid from attaching to the inner circumferential surface (in particular, the female screw 915) of the outer tube 913 of the male connector 910.

An object of the present invention is to prevent a medicinal liquid from attaching to a female screw that surrounds a male member of a male connector when an injector is connected to the male connector in a simple suspension method.

### Means for Solving Problem

A first medical liquid collection tip according to the present invention is to be detachably mounted on a tube end of a barrel of an injector. An inner circumferential surface of the tube end of the injector is a female tapered surface with an inner diameter that becomes larger as a leading end is approached. The medical liquid collection tip includes, at one end, a male member that is to be inserted into the tube end, and includes a liquid collection needle at another end. A flow path penetrates through the medical liquid collection tip in a lengthwise direction thereof and causes the male member and the liquid collection needle to be in communication. An outer circumferential surface of the male member is a male tapered surface with an outer diameter that becomes smaller as a leading end is approached, and is configured to be fluid-tightly connected to the female tapered surface of the tube end.

A second medical liquid collection tip according to the present invention is to be detachably mounted on a tube end of a barrel of an injector. The injector includes a small-diameter portion between the tube end and a liquid storing portion. The medical liquid collection tip includes, at one end, a sealing tube configured to be fluid-tightly connected to the small-diameter portion, and includes a liquid collection needle at another end. A flow path penetrates through the medical liquid collection tip in a lengthwise direction thereof and causes the sealing tube and the liquid collection needle to be in communication.

A liquid collection nozzle of the present invention includes the first or second medical liquid collection tip, and a nozzle tip that is to be detachably mounted on the medical liquid collection tip so as to cover at least a leading end of the liquid collection needle. The nozzle tip includes an opening that is provided so as to communicate with the flow path of the medical liquid collection tip when the nozzle tip is mounted on the medical liquid collection tip.

A first injector set of the present invention includes the injector and the first or second medical liquid collection tip, which can be detachably mounted on the tube end of the injector.

A second injector set of the present invention includes the injector and the liquid collection nozzle, which can be detachably mounted on the tube end of the injector.

### Effects of the Invention

The first medical liquid collection tip according to the present invention includes a male member that can be inserted into the tube end of the injector and can be fluid-tightly connected to the tube end. The second medical liquid collection tip according to the present invention includes a sealing tube that can be fluid-tightly connected to the small-diameter portion of the injector. The liquid collection nozzle according to the present invention includes a first or second liquid collection tip and a nozzle tip that is detachably mounted on the liquid collection tip. Accordingly, in the case of performing a simple suspension method, the medicinal liquid does not attach to the outer circumferential surface of the tube end if the medicinal liquid is suctioned into the injector via the liquid collection tip while the liquid collection tip is mounted on the tube end of the injector. Thereafter, when the liquid collection tip is removed from the tube end and the tube end is connected to the male connector (patient-side connector), no medicinal liquid attaches to the female screw that surrounds the male member of the male connector.

### Brief Description of Drawings

[FIG. 1] FIG. 1A is a perspective view from a male member side of a medical liquid collection tip according to Embodiment 1 of the present invention. FIG. 1B is a perspective view from a liquid collection needle side of the medical liquid collection tip according to Embodiment 1 of the present invention.
[FIG. 2] FIG. 2 is a plan view from a male member side of the medical liquid collection tip according to Embodiment 1 of the present invention.
[FIG. 3] FIG. 3A is a cross-sectional view of the medical liquid collection tip according to Embodiment 1 of the present invention, which is taken along a plane including 3A-3A in FIG. 2 and viewed in the direction of the arrows. FIG. 3B is a cross-sectional view of the medical liquid collection tip according to Embodiment 1 of the present invention, which is taken along a plane including 3B-3B in FIG. 2 and viewed in the direction of the arrows.
[FIG. 4] FIG. 4A is a perspective view showing an injector on which the medical liquid collection tip according to the present invention is to be mounted. FIG. 4B is a cross-sectional view of the injector.
[FIG. 5] FIG. 5A is a perspective view of the medical liquid collection tip according to Embodiment 1 of the present invention, which is mounted on a tube end of the injector. FIG. 5B is a cross-sectional view of the medical liquid collection tip and the injector according to Embodiment 1 of the present invention, shown in FIG. 5A.
[FIG. 6] FIG. 6 is an enlarged cross-sectional view showing a tube end of an injector and its periphery, on which the medical liquid collection tip according to Embodiment 1 of the present invention is mounted.
[FIG. 7] FIG. 7 is an enlarged perspective view showing a leading end portion of a male member of a medical liquid collection tip according to Embodiment 2 of the present invention.
[FIG. 8] FIG. 8 is an enlarged cross-sectional view showing a tube end of an injector and its periphery, on which the medical liquid collection tip according to Embodiment 2 of the present invention is mounted.
[FIG. 9] FIG. 9 is an enlarged cross-sectional view showing a tube end of an injector and its periphery, on which a conventional tip is mounted.
[FIG. 10] FIG. 10A is a perspective view from above of a medical liquid collection tip according to Embodiment 3 of the present invention. FIG. 10B is a perspective view from below of the medical liquid collection tip according to Embodiment 3 of the present invention.
[FIG. 11] FIG. 11 is a cross-sectional perspective view of the medical liquid collection tip according to Embodiment 3 of the present invention.
[FIG. 12] FIG. 12 is a plan view of the medical liquid collection tip according to Embodiment 3 of the present invention.
[FIG. 13] FIG. 13A is a perspective view from above of a nozzle tip to be mounted on the medical liquid collection tip according to Embodiment 3 of the present invention. FIG. 13B is a perspective view from below of the nozzle tip.
[FIG. 14] FIG. 14 is a cross-sectional perspective view of the nozzle tip according to Embodiment 3 of the present invention.
[FIG. 15] FIG. 15A is a perspective view of a liquid collection nozzle obtained by mounting the nozzle tip on the medical liquid collection tip in Embodiment 3 of the present invention. FIG. 15B is a cross-sectional view of the liquid collection nozzle.
[FIG. 16] FIG. 16 is an exploded perspective view of the injector, the medical liquid collection tip, and the nozzle tip in Embodiment 3 of the present invention.
[FIG. 17] FIG. 17A is a perspective view showing a state in which the medical liquid collection tip is mounted on the injector in Embodiment 3 of the present invention. FIG. 17B is a cross-sectional view of FIG. 17A.
[FIG. 18] FIG. 18A is a perspective view showing a state in which the medical liquid collection tip and the nozzle tip are mounted in sequence on the injector in Embodiment 3 of the present invention. FIG. 18B is a cross-sectional view of FIG. 18A.
[FIG. 19] FIG. 19 is a perspective view showing a state in which the injector on which the nozzle tip has been mounted is inserted into an opening of a container in Embodiment 3 of the present invention.
[FIG. 20] FIG. 20 is a cross-sectional view of another liquid collection nozzle in Embodiment 3 of the present invention.
[FIG. 21] FIG. 21A is a perspective view from above of another medical liquid collection tip according to Embodiment 3 of the present invention. FIG. 21B is a cross-sectional perspective view of another medical liquid collection tip according to Embodiment 3 of the present invention.
[FIG. 22] FIG. 22A is a perspective view of an injector of an injector provided with a conventional liquid collection tip that is used in a simple suspension method. FIG. 22B is a cross-sectional view of the injector provided with the liquid collection tip.
[FIG. 23] FIG. 23A is a perspective view of a male connector (patient-side connector) that is being considered as ISO 80369-3. FIG. 23B is a cross-sectional view of the male connector taken along a plane including the central axis of the male connector.
[FIG. 24] FIG. 24A is a perspective view of a female connector (container-side connector) that is being considered as ISO 80369-3. FIG. 24B is a cross-sectional view of the female connector taken along a plane including the central axis of the female connector.

### Description of the Invention

The first medical liquid collection tip of the present invention can furthermore include an outer tube that is separate from the male member and surrounds the male member. In this case, when the male member is inserted into the tube end of the injector, the outer tube preferably surrounds the tube end. This preferable configuration is advantageous for ensuring a favorable hygienic state of the tube end of the injector.

The sealing tube that is in communication with the flow path may protrude from the leading end of the male member. In this case, it is preferable that the sealing tube is configured to be fluid-tightly connected to the small-diameter portion between the tube end and the liquid storing portion of the barrel when the male member is inserted into the tube end. This preferable configuration is advantageous for accurately managing a medicinal liquid amount to be administered to the patient.

The male member preferably conforms to ISO 80369-3. According to this preferable configuration, the male member can be fluid-tightly connected to the injector having a tube end that includes a female connector conforming to ISO 80369-3.

The second medical liquid collection tip of the present invention can furthermore include an outer tube that is configured to surround the tube end when the sealing tube is fluid-tightly connected to the small-diameter portion. This preferable configuration is advantageous for ensuring a favorable hygienic state of the tube end of the injector.

In the first and second medical liquid collection tips of the present invention, a spiral protrusion (male screw) may be formed on the outer circumferential surface of the tube end of the injector. In one configuration example, the screw structure (e.g., the female screw) that screws onto the spiral projection is not formed on the inner circumferential surface of the outer tube. This configuration is advantageous for easily and quickly performing a task of removing the liquid collection tip from the injector. In another configuration example, the screw structure that screws onto the spiral projection is formed on the inner circumferential surface of the outer tube. This configuration is advantageous for firmly mounting the liquid collection tip on the tube end of the injector.

The first and second medical liquid collection tips of the present invention are preferably formed integrally as one part. This preferable configuration is advantageous for easily manufacturing the liquid collection tip.

The liquid collection nozzle of the present invention includes the first or second medical liquid collection tip, and a nozzle tip that is detachably mounted on the medical liquid collection tip so as to cover at least the leading end of the liquid collection needle. The nozzle tip includes an opening that is provided so as to communicate with the flow path of the medical liquid collection tip when the nozzle tip is mounted on the medical liquid collection tip. Accordingly, an injector that can be used in the simple suspension method can be used to extract breast milk if the nozzle tip is mounted on the liquid collection tip.

With the liquid collection nozzle of the present invention, an air-tight and fluid-tight seal is preferably formed between the medical liquid collection tip and the nozzle tip when the nozzle tip is mounted on the medical liquid collection tip. This preferable configuration is advantageous for efficiently extracting breast milk.

A lock mechanism for maintaining the state in which the nozzle tip is mounted on the first or second medical liquid collection tip is preferably provided on the liquid collection nozzle of the present invention. This preferable configuration is advantageous for preventing the nozzle tip from unintentionally falling off of the liquid collection tip.

A male tapered surface whose outer diameter becomes smaller as the leading end is approached may be provided on the outer circumferential surface of the nozzle tip. According to this configuration, the injector can pierce the container in a state in which the male tapered surface has been fit into the edge of the opening of the container. This is advantageous for preventing contamination of the leading end of the nozzle tip and preventing contamination and evaporation of the liquid in the container.

Hereinafter, the present invention will be described in detail by means of preferred embodiments. However, it goes without saying that the present invention is not limited to the following embodiments. In the drawings referenced in the following description, only the relevant members needed in order to describe the present invention among the members constituting the embodiment of the present invention are shown in a simplified manner for the sake of convenience in the description. Accordingly, the present invention can include any member that is not shown in the following drawings. Also, in the following drawings, the actual dimensions of members and dimensional proportions of members and the like are not necessarily rendered faithfully. In the drawings shown below, identical members are denoted by identical reference signs, and redundant description thereof is not included.

### Embodiment 1

### Configuration

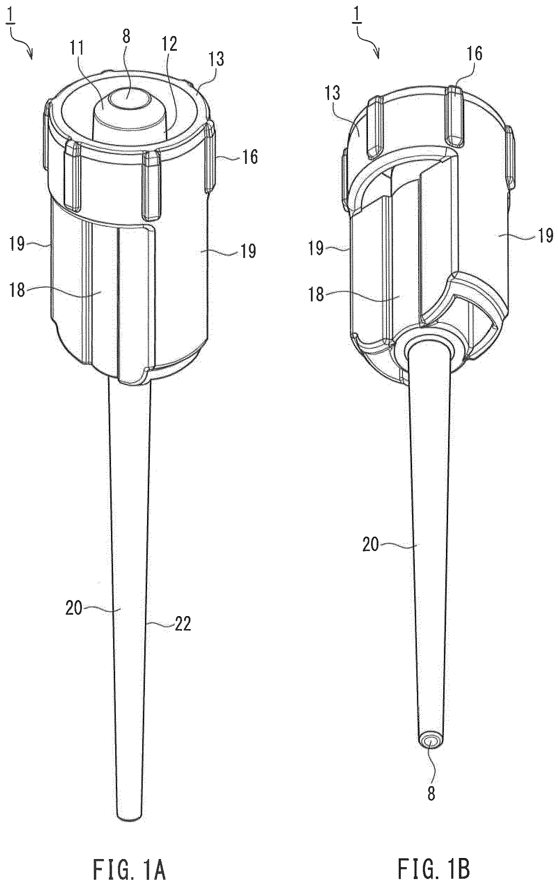

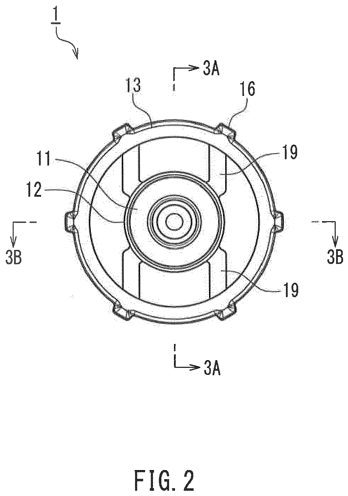

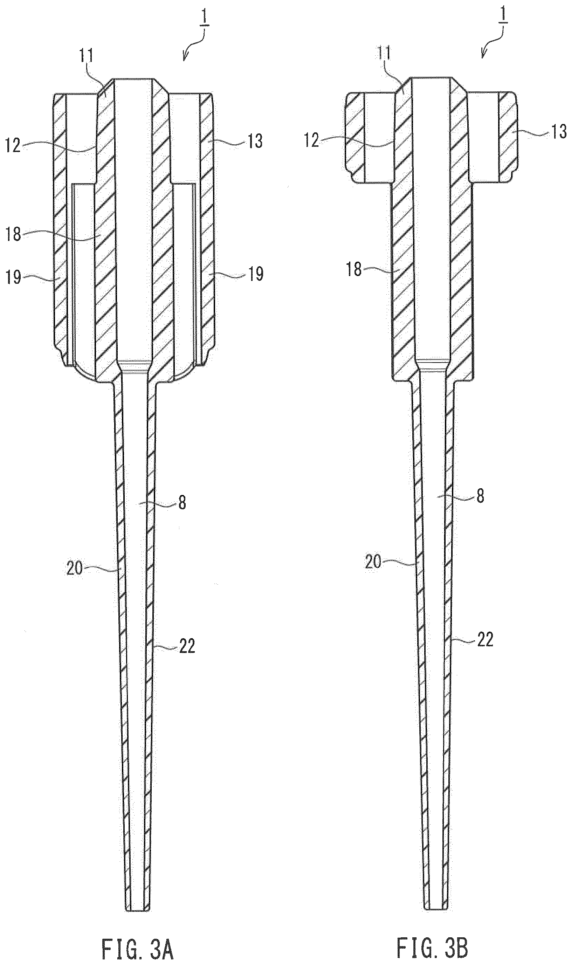

FIG. 1A is a perspective view from above of a medical liquid collection tip (hereinafter referred to as "liquid collection tip") 1 according to Embodiment 1 of the present invention. FIG. 1B is a perspective view from below of the liquid collection tip 1. FIG. 2 is a plan view of the liquid collection tip 1. FIG. 3A is a cross-sectional view of the liquid collection tip 1 taken along a plane including 3A-3A in FIG. 2 and viewed in the direction of the arrows. FIG. 3B is a cross-sectional view of the liquid collection tip 1 taken along a plane including 3B-3B in FIG. 2 and viewed in the direction of the arrows. The liquid collection tip 1 includes a male member 11 at one end and a liquid collection needle 20 at the other end. For the sake of convenience in the following description, the male member 11 side is referred to as the "upper" side of the liquid collection tip 1 and the liquid collection needle 20 side is referred to as the "lower" side of the liquid collection tip 1. Also, the direction in which the male member 11 and the liquid collection needle 20 are connected is referred to as the lengthwise direction" of the liquid collection tip 1.

The male member 11 has a hollow tube shape. The outer circumferential surface 12 of the male member 11 is a tapered surface (a conical surface, or a so-called male tapered surface) with an outer diameter that becomes smaller as the leading end is approached. An outer tube 13 surrounds the male member 11. The outer tube 13 has a hollow, approximately cylindrical shape, opposes the outer circumferential surface 12 of the male member 11, and is separated from the outer circumferential surface 12. A pair of support platforms 19 that are approximately "U"-shaped in cross-section are provided so as to protrude outwardly on the outer circumferential surface of a base portion 18, which is a portion located on the liquid collection needle 20 side with respect to the male member 11. The pair of support platforms 19 are arranged on both sides of the base portion 18. The lower end of the outer tube 13 is connected to the upper end of the outer circumferential surfaces of the support platforms 19, whereby the outer tube 13 is held to the base portion 18 via the support platforms 19.

As is shown best in FIG. 1B, the pair of support platforms 19 and the base portion 18 therebetween form a pair of approximately flat surfaces that are parallel to each other. Also, multiple ribs (ridge-shaped projections) 16 that extend in the lengthwise direction of the liquid collection tip 1 are formed at equal angular intervals on the outer circumferential surface of the outer tube 13. The pair of approximately flat surfaces and the multiple ribs 16 are effective for stably holding the liquid collection tip 1 between fingers.

The tapered surface of the outer circumferential surface 12 of the male member 11 conforms to ISO 80369-3 regarding the outer circumferential surface 912 of the male member 911 of the above-described male connector 910 (see FIGS. 23A and 23B). The inner circumferential surface of the outer tube 13 is a cylindrical surface that is coaxial with the male member 11, and the inner circumference thereof is the same as or larger than the trough diameter of the female screw 915 formed on the inner circumferential surface of the outer tube 913 of the male connector 910. For example, the male member 11 and the outer tube 13 may be the same as the male connector 910 from which the female screw 915 has been omitted.

The liquid collection needle 20 also has a hollow cylindrical shape. The outer circumferential surface 22 of the liquid collection needle 20 is a tapered surface (a male tapered surface) with an outer diameter that becomes smaller as the leading end is approached. However, the shape of the outer circumferential surface 22 is not limited thereto, and may be a cylindrical surface with a constant outer diameter in the lengthwise direction, for example.

As shown in FIGS. 3A and 3B, a flow path 8 penetrates through the liquid collection tip 1 in the lengthwise direction and openings are formed at the leading end of the male member 11 and the leading end of the liquid collection needle 20. The male member 11 and the liquid collection needle 20 are in communication via the flow path 8.

The material of the liquid collection tip 1 is not limited but is preferably a material with a shape-holding property, and furthermore, is preferably a hard material (solid material) that has a mechanical strength (rigidity) according to which deformation substantially does not occur due to an external force. For example, it is possible to use a resin material such as polypropylene (PP), polycarbonate (PC), polyacetal (POM), polystyrene, polyamide, polyethylene, rigid polyvinyl chloride, or acrylonitrile-butadiene-styrene copolymer (ABS), and among these, polypropylene (PP), polyethylene, polycarbonate (PC), and acrylonitrile-butadiene-styrene copolymer (ABS) are preferable. The entire liquid collection tip 1 can be manufactured integrally as one part overall though extrusion molding or the like using the above-described resin material.

### Method of Use

The liquid collection tip 1 is used when suctioning a medicinal liquid in a container into an injector in the above-described simple suspension method.

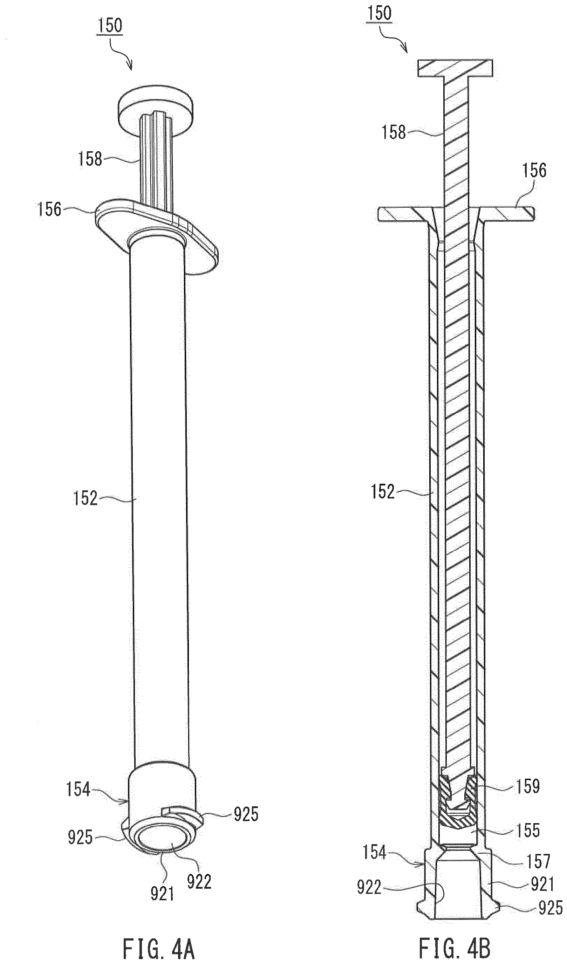

FIG. 4A is a perspective view of an injector (syringe) 150 on which the liquid collection tip 1 is to be mounted. FIG. 4B is a cross-sectional view of the injector 150. The injector 150 includes a barrel (outer tube) 152 and a plunger 158. The barrel 152 has a hollow cylindrical shape, one end thereof (upper end) is open, and a tube end (nozzle) 154 is included at the other end (lower end). The plunger 158 is inserted into the opening at the upper end of the barrel 152 so as to be able to move in and out.

The shape of the tube end of the injector 150 is markedly different from that of the injector 950 shown in FIGS. 22A and 22B. A female connector conforming to ISO 80369-3, which is the same as the female connector (container-side connector) 920 shown in FIG. 24A and 24B, is provided at the tube end (nozzle) 154 of the injector 150 so as to be connectable to the male connector (patient-side connector) 910 (see FIGS. 23A and 23B). In FIGS. 4A and 4B, members that are identical to the members shown in FIGS. 24A and 24B are denoted by identical reference signs. As shown in FIG. 4B, a small-diameter portion 157 having a smaller inner diameter than a liquid storing portion 155 and a tubular portion 921 is formed between the liquid storing portion 155 and the tubular portion 921. Here, the liquid storing portion 155 is a portion of the barrel 152 that the plunger 158 moves into and out of and is a portion that can store the medicinal liquid. The small-diameter portion 157 prevents the plunger 158 inserted into the liquid storing portion 155 from advancing into the tubular portion 921 of the tube end 154.

A gasket 159 is attached to the leading end of the plunger 158. The gasket 159 slides in the lengthwise direction on the inner circumferential surface of the barrel 152 while forming a fluid-tight seal with the inner circumferential surface of the barrel 152. A pair of finger-hooking flanges 156 protrude outward from the upper end of the barrel 152. Notches (not shown) indicating a liquid amount in the barrel 152 are provided on the outer circumferential surface of the barrel 152.

A simple suspension method using the liquid collection tip 1 and the injector 150 is performed as follows.

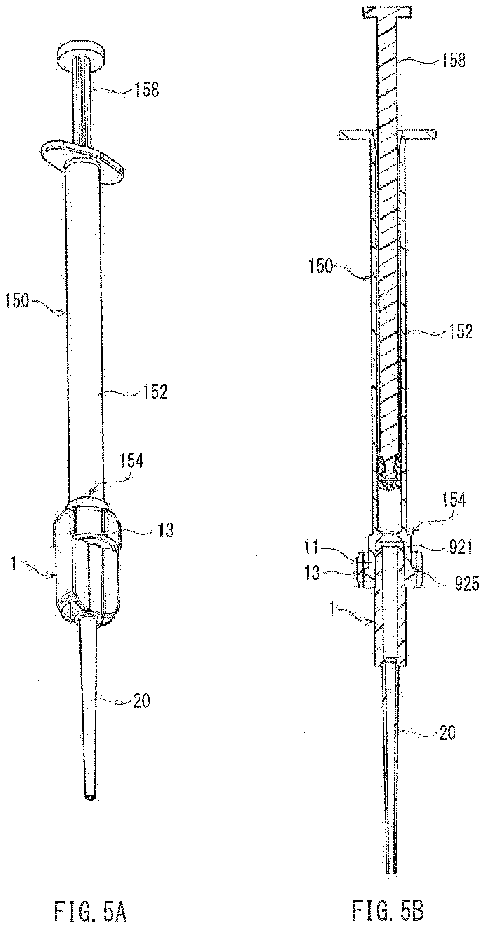

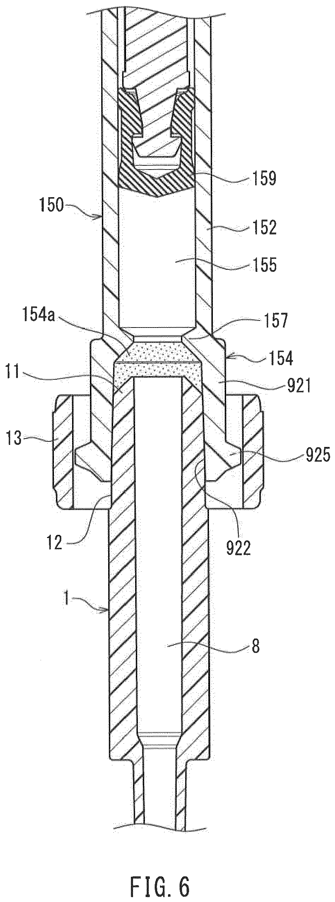

First, the liquid collection tip 1 is mounted on the tube end 154 of the injector 150. FIG. 5A is a perspective view of the liquid collection tip 1 mounted on the injector 150. FIG. 5B is a cross-sectional view of the liquid collection tip 1 mounted on the injector 150. The cross section shown in FIG. 5B is the same as the cross section shown in FIG. 3B. FIG. 6 is an enlarged cross-sectional view showing the tube end 154 shown in FIG. 5B and the periphery thereof.

The male member 11 of the liquid collection tip 1 is inserted into the tubular portion 921 of the tube end 154 of the injector 150. The outer circumferential surface 12 of the male member 11 is a male tapered surface conforming to ISO 80369-3, and the inner circumferential surface 922 of the tubular portion 921 of the injector 150 is a female tapered surface conforming to ISO 80369-3. That is, the outer circumferential surface 12 and the inner circumferential surface 922 are tapered surfaces that have the same tapering angle and diameter. Accordingly, the outer circumferential surface 12 and the inner circumferential surface 922 are in close contact, and the male member 11 and the tubular portion 921 are fluid-tightly connected. The flow path 8 of the liquid collection tip 1 and the liquid storing portion 155 of the barrel 152 are in communication.

Next, in a state in which the liquid collection tip 1 is connected to the tube end 154 of the injector 150 as shown in FIGS. 5A, 5B, and 6, the leading end of the liquid collection needle 20 is immersed in the medicinal liquid in which a tablet has been disintegrated, the plunger 158 is operated, and the medicinal liquid is suctioned into the barrel 152. The suction amount of the medicinal liquid is measured using the position of the gasket 159, which can be seen through the barrel 152, and the notches (not shown) on the barrel 152.

Next, the liquid collection tip 1 is taken off of the injector 150 (see FIGS. 4A and 4B).

Next, the tube end 154 of the injector 150 is connected to the male connector 910 (see FIGS. 23A and 23B). The male connector 910 is a patient-side connector provided on the upstream-side end of a catheter inserted into the body of the patient, or provided on the upstream-side end of an elongated tube connected to the catheter. Since the tube end 154 is the female connector 920 conforming to ISO 80369-3, the tube end 154 and the male connector 910 are connected with a fluid-tightness and a connection strength conforming to ISO 80369-3. In this state, the plunger 158 is pressed into the barrel 152 until the gasket 159 hits the small-diameter portion 157, whereby the medicinal liquid in the liquid storing portion 155 of the injector 150 is administered to the patient. Thereafter, the injector 150 is separated from the male connector 910.

### Effect

According to the present Embodiment 1, the task (suctioning task) of suctioning the medicinal liquid in the container into the injector 150 is performed with the liquid collection tip 1 mounted on the tube end 154 of the injector 150, and on the other hand, the task (administration task) of administering the medicinal liquid in the injector 150 to the patient is performed with the tube end 154 of the injector 150 connected to the male connector 910 without using the liquid collection tip 1. In the suctioning task, the leading end of the liquid collection needle 20 of the liquid collection tip 1 is immersed in the medicinal liquid. Also, the male member 11 of the liquid collection tip 1 is inserted into the tubular portion 921 of the injector 150, and the male member 11 and the tubular portion 921 are fluid-tightly connected. Accordingly, the medicinal liquid does not attach to the outer circumferential surface of the tubular portion 921, which includes the spiral projection 925. For this reason, when the tube end 154 is connected to the male connector 910 thereafter, the medicinal liquid does not attach to the inner circumferential surface (in particular, the female screw 915) of the outer tube 913 of the male connector 910. Accordingly, it is possible to prevent the male connector 910 from reaching an unhygienic state, even if the male connector 910 is left in the patient for a long time.

When the suctioning task of suctioning the medicinal liquid into the injector 150 is performed with the tube end 154 directly immersed in the medicinal liquid without mounting the liquid collection tip 1 of the present Embodiment 1 on the tube end 154, the medicinal liquid will attach not only to the inner circumferential surface of the tubular portion 921 but also to the outer circumferential surface of the tubular portion 921 including the spiral projection 925. Thereafter, when the tube end 154 is connected to the male connector 910 (see FIGS. 23A and 23B), the medicinal liquid attached to the outer circumferential surface of the tubular portion 921 attaches to the female screw 915 formed on the inner circumferential surface of the outer tube 913 of the male connector 910. If the suctioning task is performed with the liquid collection tip 1 of the present Embodiment 1 mounted on the tube end 154 of the injector 150, it is possible to reliably prevent the medicinal liquid from attaching to the outer circumferential surface of the tubular portion 921 including the spiral projection 925.

As described above, the patient-side connector is the male connector 910 shown in FIGS. 23A and 23B, and in the case of performing the simple suspension method with the patient-side connector, it is necessary to use the injector 150 (see FIGS. 4A and 4B), which has the tube end 154 conforming to International Standard ISO 80369-3, as the injector that is to connect to the patient-side connector (male connector 910). A conventional tip 940 (see FIGS. 22A and 22B) cannot be mounted on the tube end 154. The liquid collection tip 1 of the present Embodiment 1 includes the male member 11 conforming to ISO 80369-3, and thus can fluid-tightly connect to the tube end 154 of the injector 150.

The outer tube 13 of the liquid collection tip 1, which surrounds the male member 11, exhibits the following effects.

First, the outer tube 13 facilitates the task of removing the liquid collection tip 1 from the injector 150. As can be understood from FIGS. 5A and 5B, in the state in which the liquid collection tip 1 is connected to the tube end 154 of the injector 150, the outer tube 13 stores and covers the spiral projection 925 of the tubular portion 921. When the liquid collection tip 1 is to be removed from the injector 150, the barrel 152 of the injector 150 is gripped with the four fingers from the index finger to the pinky of one hand, and the thumb of that hand comes into contact with the upper-side end (injector 150 side end) of the outer tube 13 of the liquid collection tip 1. Then, when the outer tube 13 is pressed forward with the thumb, it is possible to easily cause the liquid collection tip 1 to fall off of the injector 150. Thus, with the outer tube 13, it is possible to easily and quickly perform a task of removing the liquid collection tip 1 from the injector 150 with one hand. Unlike the outer tube 913 (see FIGS. 23A and 23B) of the male connector 910 conforming to ISO 80369-3, the screw structure (female screw) that screws onto the spiral projection 925 is not formed on the inner circumferential surface of the outer tube 13 of the liquid collection tip 1. This is advantageous for easily and quickly performing a task of removing the liquid collection tip 1 from the injector 150.

Secondly, the outer tube 13 is advantageous for ensuring the hygienic state of the tube end 154 of the injector 150. As can be understood from FIG. 1A, in the state prior to the liquid collection tip 1 being mounted on the injector 150, the outer tube 13 prevents fingers of a worker or the like from touching the male member 11. Accordingly, when the liquid collection tip 1 is mounted on the injector 150 thereafter, it is possible to prevent a circumstance in which the contaminated male member 11 contaminates the tube end 154 of the injector 150. Also, in the state in which the liquid collection tip 1 is mounted on the injector 150, the outer tube 13 prevents the fingers of the worker or the like from touching the tube end 154. Accordingly, it is possible to prevent a circumstance in which the tube end 154 is contaminated. As a result of thus ensuring the hygienic state of the tube end 154, it is possible to preferably maintain the hygienic state of the male connector 910 to which the tube end 154 is to be connected thereafter, and contamination of the patient can be prevented.

### Embodiment 2

### Configuration

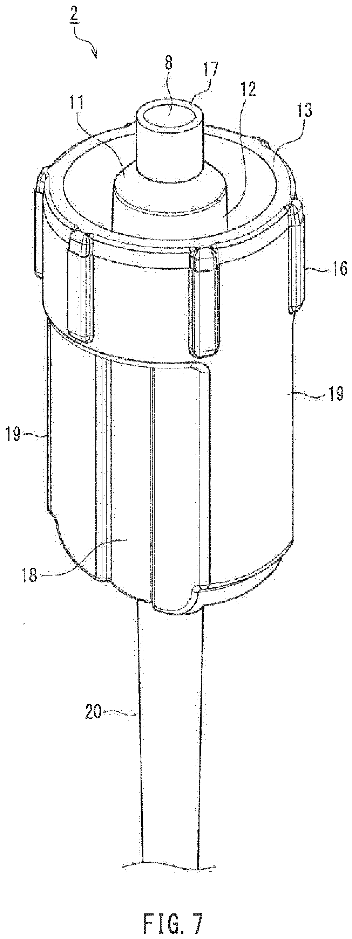

The present Embodiment 2 differs from Embodiment 1 with regard to the shape of the male member. Hereinafter, the present Embodiment 2 will be described with a focus on points of difference from Embodiment 1. FIG. 7 is an enlarged perspective view showing a leading end portion of the male member 11 of a liquid collection tip 2 according to Embodiment 2. In the present Embodiment 2, a hollow cylindrically-shaped sealing tube 17 protrudes from the leading end of the male member 11. The outer circumferential surface of the sealing tube 17 is a cylindrical surface that is coaxial with the outer circumferential surface 12 of the male member 11. The flow path 8 opens at the leading end of the sealing tube 17.

### Method of Use

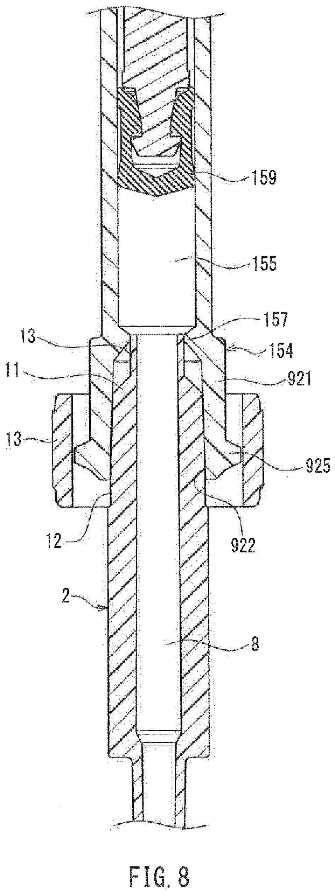

The method of use of the liquid collection tip 2 of the present Embodiment 2 is the same overall as that of the liquid collection tip 1 of Embodiment 1. FIG. 8 is an enlarged cross-sectional view of a portion at which the tube end 154 and the liquid collection tip 2 of the injector 150 are connected. Similarly to the case of Embodiment 1, the outer circumferential surface 12 of the male member 11 and the inner circumferential surface 922 of the tubular portion 921 are in close contact. In the present Embodiment 2, the sealing tube 17 at the leading end of the male member 11 is fit into the small-diameter portion 157 of the barrel 152. The outer diameter of the sealing tube 17 approximately matches the inner diameter of the small-diameter portion 157. Accordingly, when the sealing tube 17 is fit into the small-diameter portion 157 as shown in FIG. 8, a fluid-tight seal is formed between the outer circumferential surface of the sealing tube 17 and the inner circumferential end of the small-diameter portion 157. The flow path 8 of the liquid collection tip 2 and the liquid storing portion 155 of the barrel 152 are in communication.

### Effect

The medicinal liquid amount administered to the patient needs to be managed accurately. In the above-described simple suspension method, the amount of medicinal liquid is measured using the injector. However, in the case of using the conventional liquid collection tip 940 shown in FIGS. 22A and 22B or the liquid collection tip 1 of Embodiment 1, there is a problem in that the amount of medicinal liquid cannot be accurately managed in some cases, depending on the operation method of the worker.

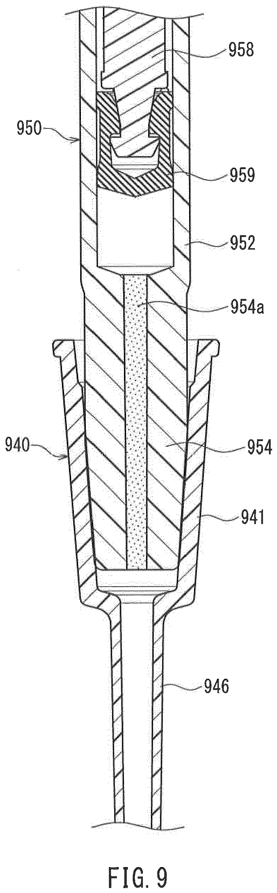

For example, in the case of performing the simple suspension method using the conventional liquid collection tip 940, as described in FIGS. 22A and 22B, the medicinal liquid is suctioned into the barrel 952 in a state in which the liquid collection tip 940 is mounted on the injector 950. The suction amount of the medicinal liquid is measured using the position of the gasket 959, which can be seen through the barrel 952, and the notches (not shown) on the barrel 952. FIG. 9 is an enlarged cross-sectional view showing the tube end 954 of the injector 950 to which the liquid collection tip 940 is mounted, and the periphery thereof. In general, the volume indicated by the notches on the barrel 952 does not include the volume of the space 954a (the portion denoted by many dots in FIG. 9) in the tube end 954. This is because the gasket 959 provided on the leading end of the plunger 958 cannot advance into the space 954a. Accordingly, the simple suspension method needs to be ended in a state in which the plunger 958 is pressed into the barrel 952 until the gasket 959 hits the deepest portion of the barrel 952, and the medicinal liquid remains in the space 954a. If the medicinal liquid that is to remain in the space 954a in the tube end 954 is also administered to the patient, the medicinal liquid will be administered in excess of the measured amount accordingly. For example, after the injector 950, which has suctioned the correct amount of medicinal liquid, is connected to the patient-side connector and the medicinal liquid in the barrel 952 has been administered to the patient, lukewarm water or the like is suctioned into the injector 950, and the medicinal liquid remaining in the space 954a is administered to the patient along with the lukewarm water, whereupon an amount of medicinal liquid that is greater than the measured amount is administered to the patient. In general, the person performing the simple suspension method is not limited to being a medical professional, and it is often the case that it is performed by a caregiver such as a family member of the patient. In such a case, there is a possibility that unsuitable medicinal liquid amount management such as that described above will be performed.

A similar problem can occur in Embodiment 1 as well. As shown in FIG. 6, the space 154a (the portion denoted by many dots in FIG. 6) is sometimes formed between the leading end of the male member 11 and the small-diameter portion 157 in the tube end 154. If the male member 11 is designed so as to be inserted deeply into the tube end 154, it is possible to make the volume of the space 154a smaller (bring the volume close to zero). However, if the space 154a is formed in the tube end 154, similarly to the description given with reference to FIG. 9, the medicinal liquid will be administered excessively when the medicinal liquid remaining in the space 154a is administered to the patient.

In contrast to this, with the liquid collection tip 2 of the present Embodiment 2, as shown in FIG. 8, when the liquid collection tip 2 is mounted on the injector 150, the tube-shaped sealing tube 17 protruding from the leading end of the male member 11 is fit into the small-diameter portion 157 of the barrel 152. Since the sealing tube 17 and the small-diameter portion 157 are fluid-tightly connected, the medicinal liquid in the liquid storing portion 155 does not pass between the sealing tube 17 and the small-diameter portion 157 to leak to the tube end 154 side. Accordingly, unlike the conventional liquid collection tip 940 and the liquid collection tip 1 of Embodiment 1, the medicinal liquid does not remain in the tube end 154 in the case of performing the simple suspension method using the liquid collection tip 2. For this reason, in the case of using the liquid collection tip 2 of the present Embodiment 2, the possibility that an amount of the medicinal liquid that is greater than the measured amount will be administered to the patient is reduced, regardless of the worker. Accordingly, the liquid collection tip 2 of the present Embodiment 2 is advantageous for accurately managing a medicinal liquid amount to be administered to the patient.

The present Embodiment 2 is the same as Embodiment 1 except for the above description. The description of Embodiment 1 is applied to the present Embodiment 2 as well.

### Embodiment 3

### Configuration

The liquid collection tip 3 of the present Embodiment 3 differs from Embodiment 2 with regard to the configuration of the portion connected to the tube end 154 of the injector 150. Furthermore, a nozzle tip 350 can be detachably mounted on the liquid collection tip 3 of the present Embodiment 3. Hereinafter, the present Embodiment 3 will be described with a focus on points of difference from Embodiments 1 and 2.

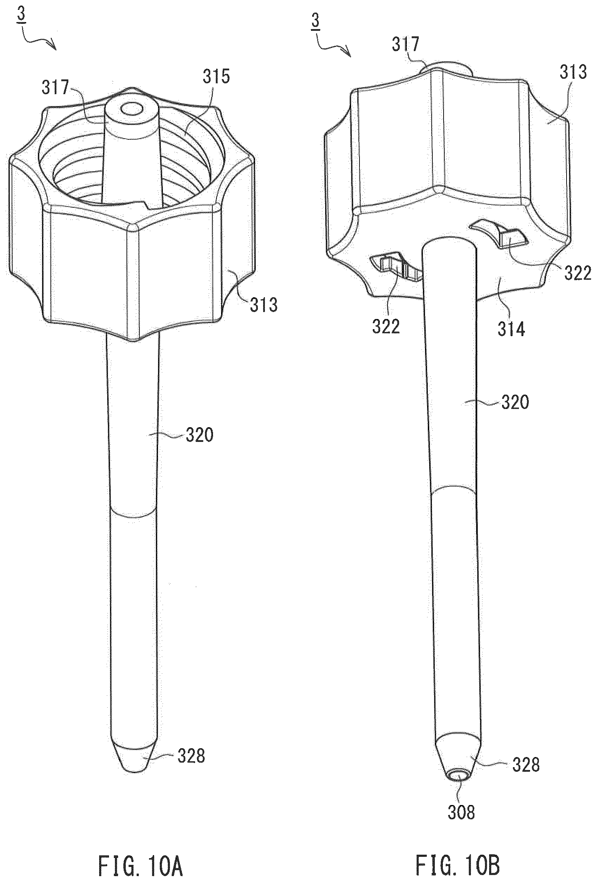

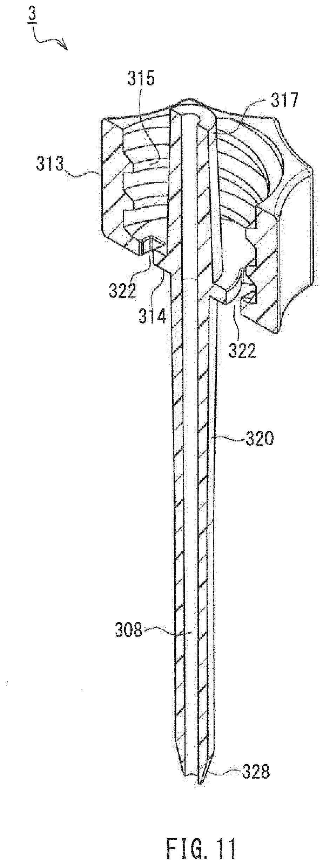

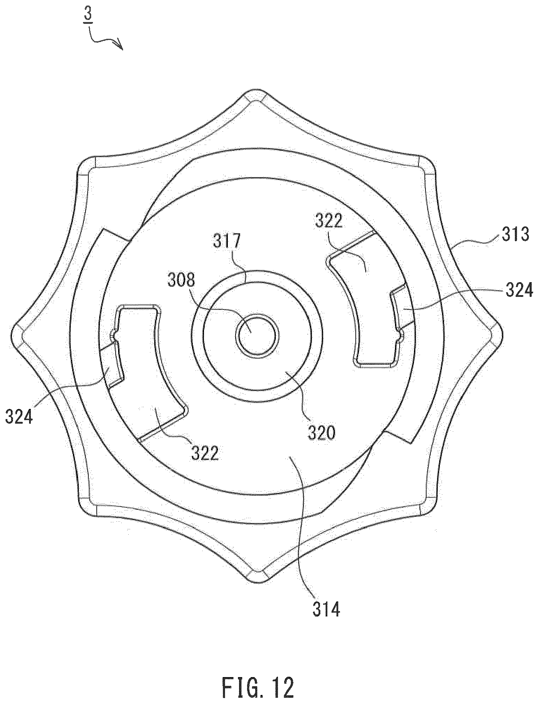

FIG. 10A is a perspective view from above of a liquid collection tip 3, and FIG. 10B is a perspective view from below of the liquid collection tip 3. FIG. 11 is a cross-sectional perspective view of the liquid collection tip 3, and FIG. 12 is a plan view of the liquid collection tip 3.

As shown in FIGS. 10A and 11, the liquid collection tip 3 includes, on its upper end, a hollow cylindrically-shaped sealing tube 317 that functions similarly to the sealing tube 17 (see FIG. 7) of Embodiment 2. The liquid collection tip 2 of Embodiment 2 included the male tapered surface 12 conforming to ISO 80369-3, which is located adjacent to the lower side with respect to the sealing tube 17, but the liquid collection tip 3 does not include this kind of male tapered surface 12. The flow path 308 penetrates through the liquid collection tip 3 in the lengthwise direction and is open at the leading end of the sealing tube 317 and the leading end of the liquid collection needle 320. The sealing tube 317 and the liquid collection needle 320 are in communication via the flow path 308.

An outer tube 313 that surrounds the liquid collection needle 320 is provided near the sealing tube 317. The liquid collection needle 320 and the outer tube 313 are joined via a bottom plate 314 that protrudes in a flange shape along the radial direction from the liquid collection needle 320. A female screw (screw structure) 315 is provided on the inner circumferential surface opposing the liquid collection needle 320 of the outer tube 313. The female screw 315 is interchangeable with the female screw 915 (see FIGS. 23A and 23B) provided on the above-described male connector 910 and conforms to ISO 80369-3. Accordingly, the female screw 315 can be screwed on the spiral projection (male screw) 925 provided on the tube end 154 of the barrel 152 conforming to ISO 80369-3. An approximately octagonal prism surface is provided on the outer circumferential surface of the outer tube 313 such that it is easy to grasp the outer tube 313 and apply a rotational force to the liquid collection tip 3. Note that the shape of the outer circumferential surface of the outer tube 313 is not limited thereto and can be changed as appropriate.

As shown in FIG. 12, two through holes 322 that are approximately L-shaped in plan view are provided on the bottom plate 314. The two through holes 322 have 180-degree rotational symmetry with respect to the central axis of the liquid collection needle 320. Inclined surfaces 324 are provided near the edges of the through holes 322 on the upper surface of the bottom plate 314. The inclined surfaces 324 are inclined so as to rise toward the clockwise direction with respect to the liquid collection needle 320.

As shown in FIG. 10A, a tapered surface (male tapered surface) 328 with an outer diameter that becomes smaller as the leading end is approached is provided on the outer circumferential surface near the leading end of the liquid collection tip 320.

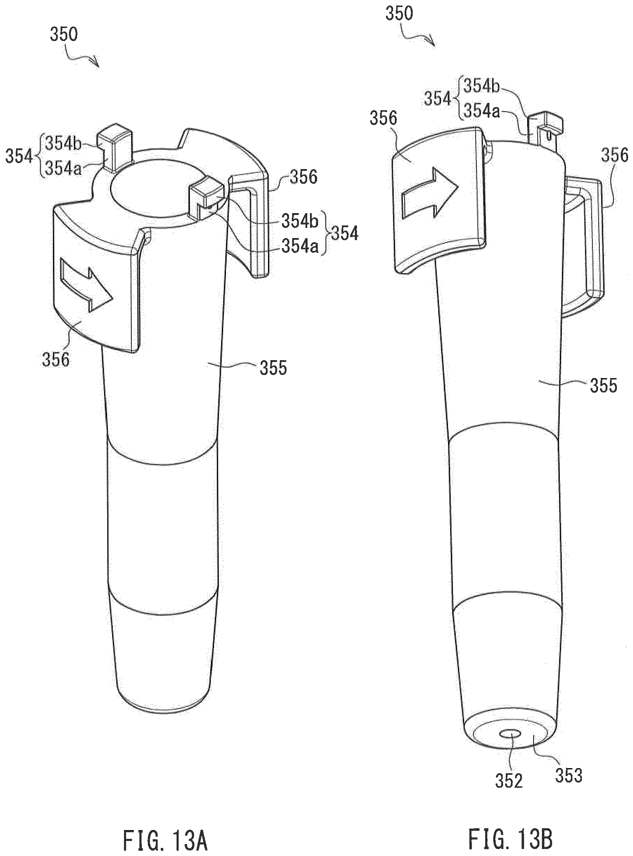

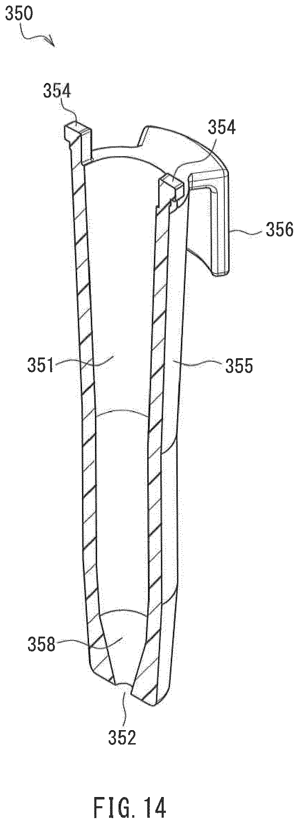

FIG. 13A is a perspective view from above of the nozzle tip 350, FIG. 13B is a perspective view from below of the nozzle tip 350, and FIG. 14 is a cross-sectional perspective view of the nozzle tip 350.

As shown in FIG. 14, the nozzle tip 350 has a hollow, approximately cylindrical shape. The inner diameter of the inner circumferential surface that defines an inner cavity 351 of the nozzle tip 350 is set to be the same as or greater than the outer diameter of the portion below the bottom plate 314 of the liquid collection needle 320 (see FIGS. 10A and 10B) so that the portion of the liquid collection needle 320 can be inserted. The inner diameter of the nozzle tip 350 is at its minimum at the opening 352 provided on the leading end thereof (the lower end). The inner diameter of the opening 352 is preferably set to be about the same as the inner diameter of the opening (see FIG. 10B) at the leading end of the flow path 308 of the liquid collection needle 320. A tapered surface (female tapered surface) 358 with an inner diameter that becomes smaller as the opening 352 is approached is provided adjacent to the opening 352 and on the upper side thereof. The female tapered surface 358 has the same tapering angle and diameter as the male tapered surface 328 (see FIG. 10A) provided near the leading end of the liquid collection needle 320.

As shown in FIG. 13A, two projections 354 are provided on the upper end of the nozzle tip 350. Each projection 354 includes a vertical portion 354a that extends upward from the upper end of the nozzle tip 350 and an engagement portion 354b that protrudes outward along the radial direction from the upper end of the vertical portion 354a. The two projections 354 have 180-degree rotational symmetry with respect to the central axis of the nozzle tip 350. Furthermore, two grasping portions 356 are provided on the nozzle tip 350. The grasping portions 356 each protrude outward along the radial direction from the upper end of the nozzle tip 350, and thereafter extend downward. The grasping portions 356 are provided in order to make it easier to apply a rotational force to the nozzle tip 350. The shapes of the grasping portions 356 are not limited to the present embodiment. For example, it is also possible to provide a regular polygonal prism surface (a regular hexagonal prism surface, a regular octagonal prism surface, or the like) on the outer circumferential surface of the nozzle tip 350 and use this as the grasping portion. The grasping portions may also be omitted.

As shown in FIG. 13B, a tapered surface (male tapered surface) 355 with an outer diameter that becomes smaller as the leading end is approached is provided on the outer circumferential surface of the nozzle tip 350. A circular plane 353 that is perpendicular to the lengthwise direction of the nozzle tip 350 is provided on the leading end of the nozzle tip 350. The outer diameter of the leading end surface 353 is larger than the outer diameter of the leading end (see FIG. 10B) of the liquid collection needle 320. The opening 352 is provided in the center of the leading end surface 353. The outer circumferential edge of the leading end surface 353 is smoothly chamfered.

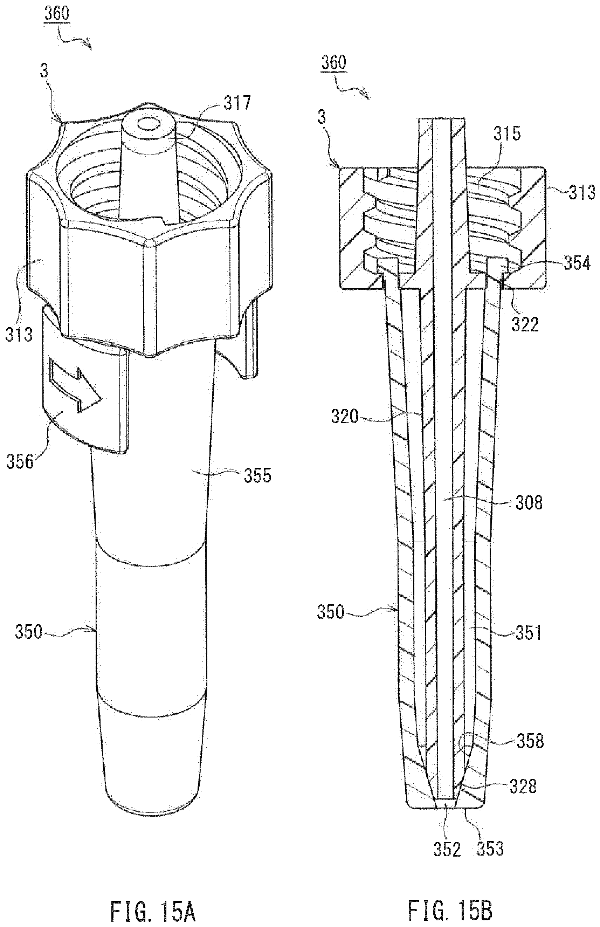

The nozzle tip 350 can be detachably mounted repeatedly on the liquid collection tip 3. FIG. 15A is a perspective view of a liquid collection nozzle 360 in which the nozzle tip 350 has been mounted on the liquid collection tip 3. FIG. 15B is a cross-sectional view of the liquid collection nozzle 360.

The nozzle tip 350 is mounted on the liquid collection tip 3 overall as follows. The liquid collection needle 320 is inserted into the inner cavity 351 of the nozzle tip 350 and the projections 354 of the nozzle tip 350 are inserted into the through holes 322 provided on the bottom plate 314 of the liquid collection tip 3. In this state, the liquid collection tip 3 and the nozzle tip 350 are rotated in mutually opposite directions (i.e., in a view from above, the liquid collection tip 3 is rotated in the counterclockwise direction with respect to the nozzle tip 350). Engagement portions 354b of the nozzle tip 350 (see FIGS. 13A and 13B) slide on the inclined surfaces 324 (see FIG. 12) of the liquid collection tip 3. Since the inclined surfaces 324 are inclined as described above, the liquid collection needle 320 moves relatively along the lengthwise direction with respect to the nozzle tip 350 such that the liquid collection needle 320 is more deeply inserted into the inner cavity 351 of the nozzle tip 350 as the nozzle tip 350 rotates with respect to the liquid collection tip 3. The nozzle tip 350 is rotated with respect to the liquid collection tip 3 until the vertical portions 354a of the projections 354 of the nozzle tip 350 come into contact with the trailing ends in the rotational direction of the edges that define the through holes 322 of the liquid collection tip 3. FIGS. 15A and 15B show this state. The male tapered surface 328 of the liquid collection needle 320 and the female tapered surface 358 of the nozzle tip 350 are fit together fluid-tightly and air-tightly. The leading end of the liquid collection needle 320 is stored in the nozzle tip 350 and is covered by the nozzle tip 350. The opening 352 on the leading end of the nozzle tip 350 and the flow path 308 of the liquid collection needle 320 are in communication.

The liquid collection tip 3 and the nozzle tip 350 can be separated by performing an operation opposite to that described above.

The nozzle tip 350 can be easily rotated relative to the liquid collection tip 3 by grasping each of the outer circumferential surface of the outer tube 313 of the liquid collection tip 3 and the grasping portion 356 of the nozzle tip 350 with different hands and applying a rotational force.

The material of the liquid collection tip 3 is not limited, and it is possible to use the same material as that of the liquid collection tip 1 described in Embodiment 1. Since the nozzle tip 350 directly touches the skin of a person, a material with a relatively low hardness is preferable, and specifically, it is possible to use a resin material such as polypropylene (PP) or polyethylene (PE). The liquid collection tip 3 and the nozzle tip 350 can be manufactured integrally as one part overall through an extrusion molding method or the like, using the above-described resin materials.

### Method of Use

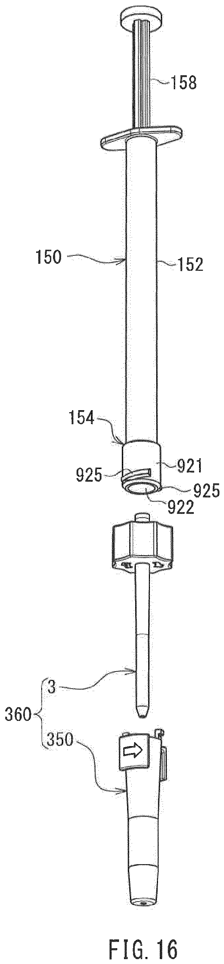

FIG. 16 is an exploded perspective view of the injector 150, the liquid collection tip 3, and the nozzle tip 350. In the present Embodiment 3, similar to Embodiments 1 and 2, the medicinal liquid in the container can be suctioned into the injector 150 in the simple suspension method when the liquid collection tip 3 has been mounted on the injector 150. Furthermore, it is possible to extract breast milk in a state in which the liquid collection tip 3 and the nozzle tip 350 have been mounted on the injector 150.

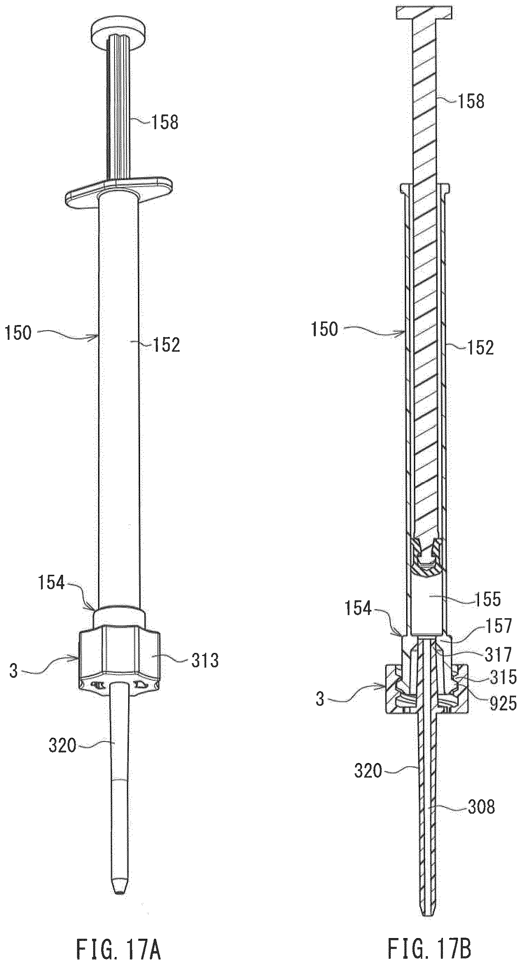

Firstly, the simple suspension method will be described. As shown in FIGS. 17A and 17B, in the case of performing the simple suspension method, the liquid collection tip 3 is mounted on the injector 150. As shown in FIG. 17B, similarly to Embodiment 2, the sealing tube 317 of the liquid collection tip 3 is fit into the small-diameter portion 157 of the barrel 152. The outer diameter of the sealing tube 317 approximately matches the inner diameter of the small-diameter portion 157. Accordingly, a fluid-tight seal is formed between the outer circumferential surface of the sealing tube 317 and the inner circumferential end of the small-diameter portion 157. The flow path 308 of the liquid collection tip 3 is in communication with the liquid storing portion 155 of the barrel 152. The female screw 315 of the liquid collection tip 3 is screwed on the spiral projection 925 of the barrel 12.

The leading end of the liquid collection needle 320 is immersed in the medicinal liquid in which a tablet has been disintegrated, the plunger 158 is operated, and the medicinal liquid is suctioned into the barrel 152. Next, the liquid collection tip 3 is taken off of the tube end 154 (see FIG. 16). Thereafter, similarly to Embodiment 1, the tube end 154 is connected to the male connector 910 (see FIGS. 23A and 23B) and the medicinal liquid in the liquid storing portion 155 is administered to the patient. Thereafter, the tube end 154 is separated from the male connector 910.

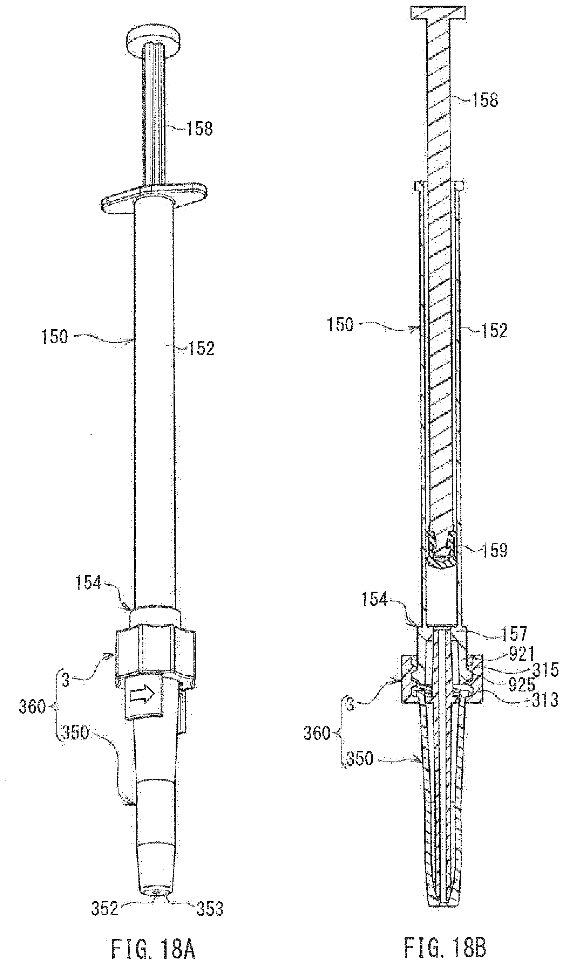

Next, breast milk extraction will be described. As shown in FIGS. 18A and 18B, breast milk is extracted in a state in which the liquid collection tip 3 and the nozzle tip 350 are mounted on the injector 150. The leading end surface 353 of the nozzle tip 350 is placed against the nipple, the plunger 158 is operated, and breast milk attached to the nipple is suctioned into the barrel 152 via the opening 352.

### Effect

According to the present Embodiment 3, in the case of performing a simple suspension method, the task (suction task) of suctioning the medicinal liquid in the container into the injector 150 is performed with the liquid collection tip 3 mounted on the tube end 154, and on the other hand, the task (administration task) of administering the medicinal liquid in the injector 150 to the patient is performed with the tube end 154 connected to the male connector 910, without using the liquid collection tip 3. Accordingly, similarly to Embodiment 1, in the suction task, the medicinal liquid does not attach to the outer circumferential surface of the tubular portion 921 including the spiral projection 925. For this reason, when the tube end 154 is connected to the male connector 910 thereafter, the medicinal liquid does not attach to the inner circumferential surface (in particular, the female screw 915) of the outer tube 913 of the male connector 910. Accordingly, it is possible to prevent the male connector 910 from reaching an unhygienic state, even if the male connector 910 is left in the patient for a long time.

When the liquid collection tip 3 is mounted on the tube end 154 (see FIGS. 17A and 17B), the sealing tube 317 of the liquid collection tip 3 and the small-diameter portion 157 of the barrel 152 are fluid-tightly connected. For this reason, similarly to Embodiment 2, the medicinal liquid in the liquid storing portion 155 does not pass between the sealing tube 317 and the small-diameter portion 157 to leak to the tube end 154 side. Accordingly, the liquid collection tip 3 of the present Embodiment 3 is advantageous for accurately managing a medicinal liquid amount to be administered to the patient.

The injector 150 (see FIGS. 18A and 18B) on which the liquid collection tip 3 and the nozzle tip 350 (i.e., the liquid collection nozzle 360) are mounted can be used preferably for breast milk extraction. In general, with breast milk extraction, breast milk is suctioned into the barrel in a state in which the tube end of the injector (syringe) is placed directly against the nipple. For example, breast milk extraction can be performed using the conventional injector 950 (see FIGS. 22A and 22B) on which the liquid collection tip 940 is not mounted. A circular plane with a relatively large area is provided on the leading end of the tube end 954, and a flow path is opened in the center thereof. The circular plane is placed against the nipple and the breast milk is suctioned. The nozzle tip 350 of the present Embodiment 3 includes a leading end surface 353 that is equal to or larger than the leading end surface of the tube end 954, and therefore the leading end surface 353 can be placed against the nipple and breast milk extraction can be performed.

In contrast to this, the leading ends of the liquid collection needles 20 and 320 of Embodiments 1 to 3 have relatively small diameters so as to be able to perform extraction without leaving even a small amount of fluid in the container. If the leading ends of the liquid collection needles 20 and 320 are directly placed against the nipple, the mother may feel pain. On the other hand, since a cavity with a large inner diameter exists in the tubular portion 921 of the tube end 154 of the injector 150 (see FIGS. 4A and 4B) from which the liquid collection tips 1, 2, and 3 have been removed, it is difficult to suction a small amount of breast milk. The nozzle tip 350 of the present Embodiment 3 has a leading end surface 353 with a relatively large diameter and large area, and therefore no pain is felt when the leading end of the nozzle tip 350 directly touches the skin. Small amounts of breast milk can be suctioned with little remaining liquid by bringing the leading end surface 353 of the nozzle tip 350 into close contact with the nipple.

Thus, according to the present Embodiment 3, it is possible to perform both the simple suspension method and breast milk extraction using the same injector 150.

With the present Embodiment 3, the nozzle tip 350 is used while mounted on the liquid collection tip 3. Unlike the present Embodiment 3, a configuration is conceivable in which a breast milk extraction nozzle having an outer shape similar to that of the nozzle tip 350 is created, and the breast milk extraction nozzle is mounted on the tube end 154 instead of the liquid collection tip 3 in the case of performing breast milk extraction. However, in this configuration, the thickness in the radial direction of the breast milk extraction nozzle needs to be increased. This kind of thick breast milk extraction nozzle generally has low resin formability. In contrast to this, the nozzle tip 350 of the present embodiment can be made thinner, and therefore has excellent resin formability.

When the nozzle tip 350 is mounted on the liquid collection tip 3, the female tapered surface 358 of the nozzle tip 350 and the male tapered surface 328 of the liquid collection tip 3 fit together near the opening 352 of the nozzle tip 350, and an air-tight and fluid-tight seal is formed between the two surfaces (see FIG. 15B). When the plunger 158 is pulled while the leading end surface 353 is pressed against the nipple, the seal prevents the external air from flowing into the flow path 308 of the liquid collection tip 3 through the gap between the nozzle tip 350 and the liquid collection needle 320 from the opening on the upper end (the outer tube 313 side) of the nozzle tip 350. For this reason, the breast milk can be suctioned into the barrel 152 through the flow path 308 of the liquid collection tip 3 from the opening 352 of the nozzle tip 350. Also, among the breast milk that flows into the opening 352 of the nozzle tip 350, the amount of breast milk that flows to the gap between the nozzle tip 350 and the liquid collection needle 320 and does not flow in the flow path 308 can be reduced. Accordingly, the seal between the liquid collection tip 3 and the nozzle tip 350 is advantageous for efficiently extracting the breast milk. The inclined surfaces 324 of the liquid collection tip 3 (see FIG. 12) are advantageous for improving the sealing property between the liquid collection tip 3 and the nozzle tip 350.

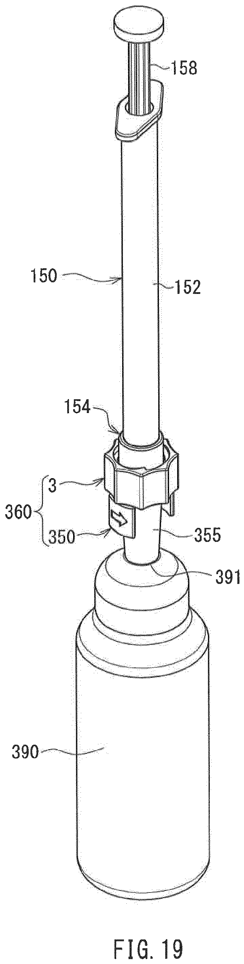

The male tapered surface 355 is provided on the outer circumferential surface of the nozzle tip 350 (see FIG. 13A). For this reason, as shown in FIG. 19 for example, the injector 150 on which the nozzle tip 350 has been mounted can pierce the open opening 391 of the container (e.g., a distilled water container) 390. The male tapered surface 355 of the nozzle tip 350 is fit into the edge of the opening 391 of the container 390, and the opening 391 is closed by the nozzle tip 350. Temporarily leaving the injector 150 and the container 390 in this state is advantageous for preventing contamination of the leading end of the nozzle tip 350 and preventing contamination and evaporation of the liquid (e.g., the distilled water) in the container 390. However, in the present invention, the male tapered surface 355 is not essential.

The liquid collection tip 3 includes the outer tube 313 (see FIG. 10A). Similarly to the outer tube 13 of Embodiment 1, the outer tube 313 is advantageous for ensuring the hygienic state of the tube end 154 of the injector 150. Accordingly, the hygienic state of the male connector 910 to which the tube end 154 is connected can be kept favorable, and contamination of the patient can be prevented. Also, the worker can attach/detach the liquid collection tip 3 to/from the tube end 154 of the injector 150 without touching the liquid collection needle 320 by holding the outer circumferential surface of the outer tube 313 of the liquid collection tip 3. The task of mounting the liquid collection nozzle 360 (see FIG. 15A) in which the nozzle tip 350 has been mounted on the liquid collection needle 320 on the tube end 154 of the injector 150 can also be performed by similarly holding the outer circumferential surface of the outer tube 313.

The female screw 315 that can be screwed onto the spiral projection 925 of the injector 150 is provided on the outer tube 313. For this reason, although the liquid collection tip 3 does not include the male tapered surface 12 (see FIGS. 1A and 7) that fits into the inner circumferential surface 922 of the tubular portion 921 of the barrel 152, which is provided in the liquid collection tips 1 and 2 of Embodiments 1 and 2, it is possible to firmly mount the liquid collection tip 3 on the barrel 152.

The female screw 315 provided in the liquid collection tip 3 and the spiral projection 925 provided in the injector 150 are so-called right screws. In contrast to this, the structure for engaging the projections 354 of the nozzle tip 350 and the bottom plate 314 of the liquid collection tip 3 is configured such that when the liquid collection tip 3 is rotated in the counterclockwise direction with respect to the nozzle tip 350 in a view from above, engagement occurs, and when the liquid collection tip 3 is rotated in the clockwise direction with respect to the nozzle tip 350, the engagement is canceled, and the relationship between the engagement, the canceling thereof, and the rotation directions is the same as that of a so-called left screw. Accordingly, in the state in which the liquid collection tip 3 and the nozzle tip 350 are mounted on the injector 150 as shown in FIGS. 18A and 18B, when each of the injector 150 and the nozzle tip 350 are grasped with a different hand and the injector 150 is rotated in the counterclockwise direction with respect to the nozzle tip 350 in a view from the injector 150 side, the nozzle tip 350 can be separated from the liquid collection tip 3 without loosening the screwing of the spiral projection 925 of the injector 150 and the female screw 315 of the liquid collection tip 3. Thus, by providing the engagement structure conforming to a left screw between the liquid collection tip 3 and the nozzle tip 350, even if one of the injector 150 and the outer tube 313 of the liquid collection tip 3 is grasped with one hand in the state shown in FIGS. 18A and 18B, the nozzle tip 350 can be reliably removed from the liquid collection tip 3 if the nozzle tip 350 is grasped with the other hand and rotated as described above.

The above-described Embodiment 3 is merely an example. The configuration of the above-described Embodiment 3 of the present invention can be modified as needed.

For example, as a lock mechanism for stably maintaining the state in which the nozzle tip 350 is mounted on the liquid collection tip 3, the above-described Embodiment 3 included an engagement structure in which the engagement portions 354b of the nozzle tip 350 are engaged with the bottom plate 314 of the liquid collection tip 3. However, the lock mechanism is not limited to this kind of engagement structure, and any configuration can be employed.

For example, the lock mechanism may be a screw structure. In an example, it is possible to provide a spiral projection (e.g., a male screw) on the outer circumferential surface of the liquid collection needle 320 and to provide a female screw that screws on the spiral projection on the inner circumferential surface near the upper end of the nozzle tip 350. In another example, the outer tube 313 of the liquid collection tip 3 may extend below the bottom surface 314, a female screw may be provided on the inner circumferential surface of the extended outer tube 313, and a screw projection (e.g., a male screw) that screws into the female screw may be provided on the outer circumferential surface near the upper end of the nozzle tip 350. The screw structure can be advantageous for improving the sealing property between the liquid collection tip 3 and the nozzle tip 350. The fact that the screw structure constituting the lock mechanism is configured to conform to a left screw is advantageous for reliably removing the nozzle tip 350 from the liquid collection tip 3 without loosening the screwing between the spiral projection 925 and the female screw 315 in a state in which the liquid collection tip 3 and the nozzle tip 350 are mounted on the injector 150 as shown in FIGS. 18A and 18B, similarly to the above-described engagement structure of Embodiment 3.

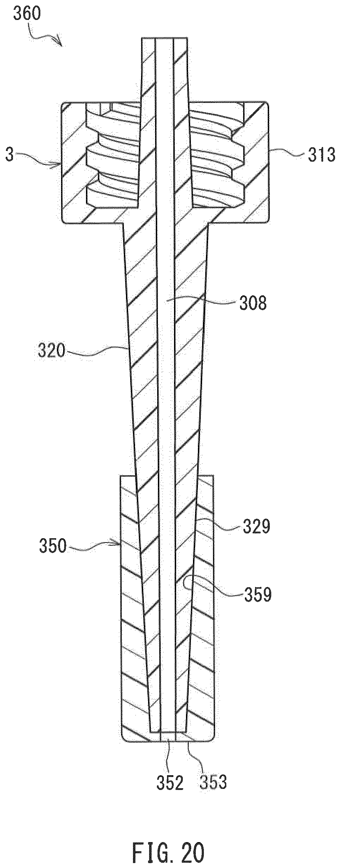

Alternatively, the above-described lock mechanism may be omitted. For example, as shown in FIG. 20, a tapered surface (male tapered surface) 329 with an outer diameter that becomes smaller as the leading end is approached is provided on the outer circumferential surface of the liquid collection needle 320, and a female tapered surface 359 with the same diameter and tapering angle as the male tapered surface 329 is provided on the inner circumferential surface of the nozzle tip 350. It is possible to stably hold the nozzle tip 350 on the liquid collection needle 320 with the frictional force between the male tapered surface 329 and the female tapered surface 359 when they are fit together. An air-tight and fluid-tight seal is formed between the male tapered surface 329 and the female tapered surface 359. In this case, the nozzle tip 350 may be constituted by a soft material that deforms relatively easily, such as a material having rubber elasticity (also referred to as an elastomer), for example, a rubber such as natural rubber, isoprene rubber, or silicone rubber, or a thermoplastic elastomer such as styrene-based elastomer, olefin-based elastomer, or polyurethane-based elastomer.

In the above-described Embodiment 3, an air-tight and fluid-tight seal was formed between the male tapered surfaces 328 and 329 of the liquid collection tip 3 and the female tapered surfaces 358 and 359 of the nozzle tip 350, but the seal between the liquid collection tip 3 and the nozzle tip 350 may be formed by surfaces other than the two fit-together tapered surfaces. For example, the air-tight and fluid-tight seal between the leading end surface (surface surrounding the opening on the leading end side of the flow path 308) of the liquid collection needle 320 and the inner surface (surface on the side opposite to that of the plane 353) of the leading end of the nozzle tip 350 may be formed by bringing them into contact in the lengthwise direction of the liquid collection needle 320.

The leading end surface 353 of the nozzle tip 350 does not need to be a precise flat surface. For example, it may protrude or be recessed in a dome shape. However, it is preferable that the corners are chamfered in a round shape so that no sharp edges are included.

The nozzle tip 350 that covers the leading end of the liquid collection needle 320 described in the present Embodiment 3 may be applied to the liquid collection tips 1 and 2 of Embodiments 1 and 2.

The above-described Embodiments 1 to 3 are merely examples. The present invention is not limited to the above-described Embodiments 1 to 3 and can be modified as needed.

With the liquid collection tips 1 and 2 of Embodiments 1 and 2, the outer tube 13 was fixed to the pair of support platforms 19, but the method of fixing the outer tube 13 is not limited to this, and any method can be used thereas. For example, similarly to the bottom plate 314 (see FIGS. 10B and 11) of the liquid collection tip 3 of Embodiment 3, or the bottom plate 914 (see FIG. 23B) of the male connector 910, a bottom plate that protrudes along the radial direction may be provided at the base end (or the base portion 18) of the male member 11, and the outer tube 913 may be fixed to the outer circumferential edge of the bottom plate. In this case, the pair of support platforms 19 can be omitted.

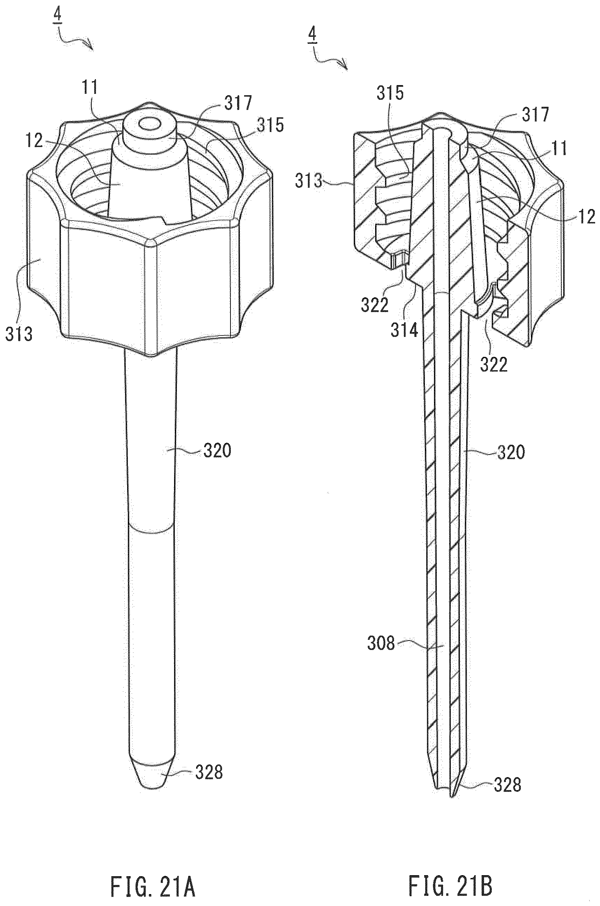

The liquid collection tip 3 of Embodiment 3 may include a fitting shape that fits into the inner circumferential surface 922 of the tubular portion 921 of the barrel 152. FIGS. 21A and 21B show an example of this kind of liquid collection tip. The liquid collection tip 4 differs from the above-described liquid collection tip 3 in that the liquid collection tip 4 is provided with the male member 11 whose outer circumferential surface 12 is a male tapered surface conforming to ISO 80369-3, similarly to the liquid collection tips 1 and 2 of Embodiments 1 and 2. The liquid collection tip 4 is detachably mounted on the tube end 154 of the barrel 152, similarly to the liquid collection tip 3. The outer circumferential surface 12 is fluid-tightly connected to the inner circumferential surface 922 of the tubular portion 921 of the barrel 152. Also, the sealing tube 317 is fluid-tightly connected to the small-diameter portion 157 of the barrel 152. The liquid collection tip 4 can be used similarly to the liquid collection tip 3 and exhibits the same effects as the liquid collection tip 3.

In Embodiments 1 and 2, the outer tube 13 may include a female screw conforming to ISO 80369-3, which is similar to the female screw 315 of Embodiment 3. Alternatively, the outer tube 13 can also be omitted.

In Embodiment 3, the female screw 315 provided on the inner circumferential surface of the outer tube 313 can be omitted. Furthermore, it is also possible to omit the female screw 315 and the outer tube 313.

The liquid collection tips 1 to 4 of the above-described Embodiments 1 to 3 were each formed integrally as one part overall, but the liquid collection tip of the present invention may also be constituted by combining two or more parts created separately. For example, the liquid collection needles 20 and 220 may be created with a soft material that deforms relatively easily (e.g., a material having rubber elasticity), and the portions other than the liquid collection needles 20 and 220 may be created with a hard material. The liquid collection needles 20 and 220 and the other portions may be connected with a soft tube.

In Embodiments 2 and 3, the shapes of the outer circumferential surfaces of the sealing tubes 17 and 317 are not limited to being cylindrical surfaces. The shapes of the sealing tubes 17 and 317 are arbitrary, as long as it is possible to fluid-tightly connect to the small-diameter portion 157. For example, the outer circumferential surfaces of the sealing tubes 17 and 317 may be male tapered surfaces (conical surfaces) with outer diameters that become smaller as the leading end is approached. Instead of fitting the sealing tubes 17 and 317 in the small-diameter portion 157, the sealing tubes 17 and 317 may be fluid-tightly connected to the small-diameter portion 157 by coming into contact with the small-diameter portion 157, for example. Alternatively, the sealing tubes 17 and 317 may be fluid-tightly connected to the small-diameter portion 157 by forming a tube-shaped projection that protrudes downward (toward the liquid collection tip) on the small-diameter portion 157 and fitting the tube-shaped projection into the sealing tubes 17 and 317.

Although a case was described in which a simple suspension method is performed using the liquid collection tips 1 to 4 in the above-described Embodiments 1 to 3, the liquid collection tip of the present invention can be used also to suction any liquid (water, medicinal liquid, blood, etc.) into the injector using a method other than the simple suspension method.

### Industrial Applicability

The present invention, although not limited, can be used widely in the field of medicine as an apparatus for suctioning any liquid (water, medicinal liquid, breast milk, blood, etc.) into an injector. In particular, the present invention can be used preferably in the case of performing a simple suspension method.

### List of Reference Numerals

- 1, 2, 3, 4: Medical liquid collection tip
- 8, 308: Flow path
- 11: Male member
- 12: Outer circumferential surface of male member (male tapered surface)
- 13, 313: Outer tube
- 315: Female screw (screw structure)
- 17, 317: Sealing tube
- 20, 320: Liquid collection needle
- 150: Injector
- 152: Barrel
- 154: Tube end
- 155: Liquid storing portion
- 157: Small-diameter portion
- 350: Nozzle tip
- 352: Opening of nozzle tip
- 354: Projection (lock mechanism)
- 355: Male tapered surface of nozzle tip
- 360: Liquid collection nozzle
- 922: Male tapered surface
- 925: Male screw (spiral projection)

## Claims

1. A medical liquid collection tip to be detachably mounted on a tube end of a barrel of an injector, wherein
an inner circumferential surface of the tube end of the injector is a female tapered surface with an inner diameter that becomes larger as a leading end is approached,
the medical liquid collection tip includes, at one end, a male member that is to be inserted into the tube end, and includes a liquid collection needle at another end,
a flow path penetrates through the medical liquid collection tip in a lengthwise direction thereof and causes the male member and the liquid collection needle to be in communication, and
an outer circumferential surface of the male member is a male tapered surface with an outer diameter that becomes smaller as a leading end is approached, and is configured to be fluid-tightly connected to the female tapered surface of the tube end.

2. The medical liquid collection tip according to claim 1, further comprising an outer tube that is separate from the male member and surrounds the male member,
wherein the outer tube surrounds the tube end when the male member is inserted into the tube end of the injector.

3. The medical liquid collection tip according to claim 1 or 2, wherein a sealing tube that is in communication with the flow path protrudes from the leading end of the male member, and
when the male member is inserted into the tube end, the sealing tube is fluid-tightly connected to a small-diameter portion between the tube end of the barrel and a liquid storing portion.

4. The medical liquid collection tip according to any one of claims 1 to 3, wherein the male member conforms to ISO 80369-3.

5. A medical liquid collection tip to be detachably mounted on a tube end of a barrel of an injector, wherein
the injector includes a small-diameter portion between the tube end and a liquid storing portion,
the medical liquid collection tip includes, at one end, a sealing tube configured to be fluid-tightly connected to the small-diameter portion, and includes a liquid collection needle at another end, and
a flow path penetrates through the medical liquid collection tip in a lengthwise direction thereof and causes the sealing tube and the liquid collection needle to be in communication.

6. The medical liquid collection tip according to claim 5, further comprising an outer tube that is configured to surround the tube end when the sealing tube is fluid-tightly connected to the small-diameter portion.

7. The medical liquid collection tip according to claim 2 or 6, wherein a spiral projection is formed on the outer circumferential surface of the tube end of the injector, and
a screw structure that screws onto the spiral projection is not formed on the inner circumferential surface of the outer tube.

8. The medical liquid collection tip according to claim 2 or 6, wherein a spiral projection is formed on the outer circumferential surface of the tube end of the injector, and
a screw structure that screws onto the spiral projection is formed on the inner circumferential surface of the outer tube.

9. The medical liquid collection tip according to any one of claims 1 to 8, wherein the medical liquid collection tip is formed integrally as one part.

10. A liquid collection nozzle comprising:
the medical liquid collection tip according to any one of claims 1 to 9; and
a nozzle tip that is to be detachably mounted on the medical liquid collection tip so as to cover at least a leading end of the liquid collection needle,
wherein the nozzle tip includes an opening that is provided so as to communicate with the flow path of the medical liquid collection tip when the nozzle tip is mounted on the medical liquid collection tip.

11. The liquid collection nozzle according to claim 10, wherein when the nozzle tip is mounted on the medical liquid collection tip, an air-tight and fluid-tight seal is formed between the medical liquid collection tip and the nozzle tip.

12. The liquid collection nozzle according to claim 10 or 11, wherein a lock mechanism for maintaining a state in which the nozzle tip is mounted on the medical liquid collection tip is provided.

13. The liquid collection nozzle according to any one of claims 10 to 12, wherein a male tapered surface with an outer diameter that becomes smaller as a leading end is approached is provided on the outer circumferential surface of the nozzle tip.

14. An injector set comprising the injector and the medical liquid collection tip according to any one of claims 1 to 9, which can be detachably mounted on the tube end of the injector.

15. An injector set comprising the injector and the liquid collection nozzle according to any one of claims 10 to 13, which can be detachably mounted on the tube end of the injector.
